# EUROPEAN PATENT APPLICATION

(11) **EP 4 328 927 A1**
(43) Date of publication of application: **28.02.2024**
(21) Application number: 22816494.3
(22) Date of filing: 03.06.2022
(51) Int. Cl.: G16H 20/70, G16H 10/20, G16H 50/30, G16H 50/20, A61B 5/16

(54) **SERVER FOR READING RESULT OF PERFORMING NEUROPSYCHOLOGICAL TEST, AND OPERATION METHOD THEREOF**

(30) Priority: 04.06.2021 KR 20210072928
(71) Applicant: Plan B4U Co., Ltd., Seongnam-si, Gyeonggi-do 13605 (KR)
(72) Inventor: KIM, Ki Woong, Seoul 06718 (KR)
(74) Representative: Mewburn Ellis LLP
(86) International application number: PCT/KR2022/007907
(87) International publication number: WO 2022/255835

(57) **Abstract**

According to an embodiment, provided is a method of performing a neuropsychological test including: an operation of acquiring one or more test items and one or more test categories including the one or more test items; an operation of acquiring an execution result of each of the one or more test items for one testee; an operation of converting the execution result into a scoring score for each of the one or more test items; an operation of matching each of the one or more test items to one or more of a plurality of cognitive ability categories; an operation of calculating an evaluation score for each of the plurality of cognitive ability categories, on the basis of the scoring scores of the one or more test items matching each of the plurality of cognitive ability categories; and an operation of generating a cognitive ability interpretation result on the basis of the calculated evaluation score.

## Description

### Technical Field

The following embodiments relate to a server for interpreting neuropsychological test results and an operation method thereof.

### Background Art

Neuropsychological tests are tests that comprehensively evaluate overall cognitive functions, and configure information necessary for early diagnosis, differential diagnosis, and rehabilitation of treatment according to cognitive areas. According to types of neuropsychological tests, the corresponding cognitive areas include combinations of attention and concentration, language and related functions, visuospatial function, memory and related functions, frontal functions including executive function, praxis, perception, instrumental and basic activities of daily living, global cognition, global severity of dementia, subjective cognitive complaints, and emotion that may affect cognitive functions. Neuropsychological tests have been developed mainly in the fields of neuropsychiatry, neurology, and neurosurgery to evaluate the status, severity, type, cause, and the like of structural or functional damage to the human brain, and have been used for screening, diagnosis, progress tracking, treatment response evaluation, insurance benefit eligibility evaluation, psychiatric evaluation, and the like of various mental disorders and neurological diseases.

### Detailed Description of the Disclosure

### Technical Problem

A server capable of interpreting neuropsychological test results and an operation method thereof are provided.

Problems to be solved by the present disclosure are not limited to the problems mentioned above, and other problems and advantages of the present disclosure that are not mentioned may be understood through the following description and may be understood more clearly by embodiments of the present disclosure. In addition, it may be understood that the problems and advantages to be solved by the present disclosure may be implemented by means and combinations thereof defined in claims.

### Technical Solution

According to an embodiment of the present disclosure, provided is a method of interpreting neuropsychological test results including: an operation of acquiring one or more test items and one or more test categories including the one or more test items; an operation of acquiring an execution result of each of the one or more test items for one testee; an operation of converting the execution result into a scoring score for each of the one or more test items; an operation of matching each of the one or more test items to one or more of a plurality of cognitive ability categories; an operation of calculating an evaluation score for each of the plurality of cognitive ability categories, on the basis of the scoring scores of the one or more test items matching each of the plurality of cognitive ability categories; and an operation of generating a cognitive ability interpretation result on the basis of the calculated evaluation score.

According to an embodiment, the operation of calculating the evaluation score may include calculating the evaluation score by adding up all the scoring scores of the one or more test items matching to the cognitive ability categories.

According to an embodiment, the operation of calculating the evaluation score may include acquiring a weight of a test category for each of the cognitive ability categories, and on the basis of the weight of the test category to which the test items belong, calculating, as the evaluation score for each of the cognitive ability categories, a value obtained by reflecting the weight in the scoring scores of the test items and adding up the scoring scores.

According to an embodiment, the operation of generating the cognitive ability interpretation result may include determining a level of cognitive impairment on the basis of the evaluation score for each of the cognitive ability categories and generating an interpretation statement corresponding to the level of the cognitive impairment.

According to an embodiment, the operation of generating the cognitive ability interpretation result may include acquiring one or more pieces of disease information on the basis of the evaluation score for each of the cognitive ability categories and generating the interpretation statement on the basis of the one or more pieces of disease information.

According to an embodiment, the operation of generating the cognitive ability interpretation result may include: an operation of acquiring priorities for the respective test categories of the one or more test items; and an operation of generating the cognitive ability interpretation result on the basis of the priorities for the respective test categories.

According to another embodiment of the present disclosure, provided is a server for interpreting neuropsychological test results including: a processor; and memory, wherein the processor is configured to acquire one or more test items and one or more test categories including the one or more test items, acquire an execution result of each of the one or more test items for one testee, convert the execution result into a scoring score for each of the one or more test items, match each of the one or more test items to one or more of a plurality of cognitive ability categories, calculate an evaluation score for each of the plurality of cognitive ability categories on the basis of the scoring scores of the one or more test items matching the plurality of cognitive ability categories, and generate a cognitive ability interpretation result on the basis of the calculated evaluation score.

Additionally, provided is a computer-readable recording medium having recorded thereon a program for executing the method on a computer.

### Advantageous Effects of Disclosure

According to the problem-solving means of the present disclosure described above, a test time may be reduced by automatically performing an interpretation operation of a neuropsychological test.

Also, according to the problem-solving means of the present disclosure, by reflecting test results for respective items in a complex way, errors or omissions in neuropsychological test interpretation may be reduced and interpretation deviations between testers may be reduced.

In addition, according to the problem-solving means of the present disclosure, when answers to tests or test items within the same cognitive area conflict with one another, the conflicting tests or test items may be interpreted by prioritizing interpretations.

### Brief Description of Drawings

FIG. 1 is a system diagram including a user terminal 1000 and a server 2000, according to an embodiment.
FIG. 2 is a diagram illustrating a neuropsychological test sequence according to an embodiment.
FIG. 3 is a view illustrating an evaluation table including a plurality of test categories and a plurality of test items included in the test categories, according to an embodiment.
FIG. 4 is a view illustrating converting execution results of test items into scoring scores, according to an embodiment.
FIG. 5 is a view illustrating matching test items to cognitive ability categories measured by the corresponding items, according to an embodiment.
FIG. 6 is a view illustrating reflecting a weight of each test category in an evaluation score, according to an embodiment.
FIG. 7 is a view illustrating generating an interpretation statement on the basis of priority, according to an embodiment.
FIG. 8 is a flowchart sequentially illustrating a neuropsychological test method according to an embodiment.
FIG. 9 is a block diagram of a server according to an embodiment.

### Mode of Disclosure

Advantages and features of the present disclosure, and methods of achieving the same will become clear with reference to the detailed description of embodiments taken in conjunction with the accompanying drawings. However, the present disclosure is not limited to the embodiments presented below, but may be implemented in various different forms, and should be understood to include all modifications, equivalents, and alternatives included in the spirit and technical scope of the present disclosure.

The terms used herein are only used to describe particular embodiments, and are not intended to limit the present disclosure. As used herein, the singular forms are intended to include the plural forms as well, unless the context clearly indicates otherwise. It should be understood that the terms "comprise", "comprising", "have", and/or "having," when used herein, specify the presence of stated features, integers, steps, operations, elements, and/or components, but do not preclude the presence or addition of one or more other features, integers, steps, operations, elements, components, and/or groups thereof.

Some embodiments of the present disclosure may be represented as functional block structures and various processing operations. Some or all of these functional blocks may be implemented as a varying number of hardware and/or software components that perform particular functions. For example, functional blocks of the present disclosure may be implemented by one or more microprocessors, or may be implemented by circuit components for a certain function. Also, for example, the functional blocks of the present disclosure may be implemented in various programming or scripting languages. The functional blocks may be implemented as an algorithm running on one or more processors. Also, the present disclosure may employ related art for electronic environment configuration, signal processing, and/or data processing. The terms such as "mechanism," "element," "means," and "component" may be used broadly, and are not limited to mechanical and physical components.

In addition, connecting lines or connecting members between components shown in the drawings are only examples of functional connections and/or physical or circuit connections. In an actual apparatus, connections between components may be represented by various functional connections, physical connections, or circuit connections that may be replaced or added.

Hereinafter, activities performed by a user may refer to activities performed by the user through a user terminal. As an example, a command corresponding to an activity performed by the user may be input into the user terminal through an input device (e.g., a keyboard, a mouse, or the like) embedded in or additionally connected to the user terminal. As another example, a command corresponding to an activity performed by the user may be input into the user terminal through a touch screen of the user terminal. Here, the activity performed by the user may include a certain gesture. For example, the gesture may include tap, touch and hold, double tap, drag, panning, flick, drag and drop, or the like.

Hereinafter, the present disclosure is described in detail with reference to the attached drawings.

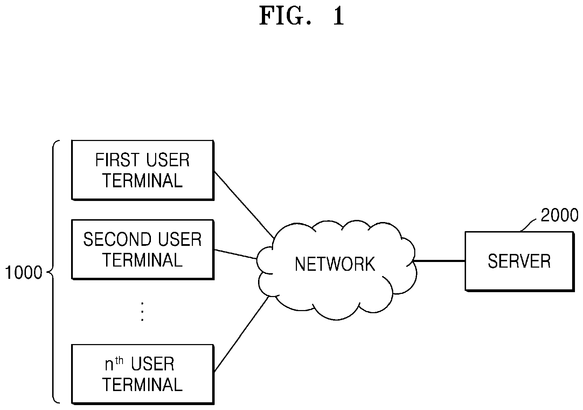

FIG. 1 is a system diagram including a user terminal and a server, according to an embodiment.

A system according to an embodiment may include a plurality of user terminals 1000 and a server 2000.

In an embodiment, the user terminal 1000 may transmit and receive data to and from the server 2000 through software, an application, and a web browser installed on the user terminal 1000. However, the method of connecting to the server 2000 is not limited thereto. As an example, the user terminal 1000 may be a terminal installed with software through which neuropsychological test scoring results may be input. In this case, a scorer may input a scoring score into the user terminal 1000, and the user terminal 1000 may transmit the scoring score to the server 1000. As another example, the user terminal 1000 may be an apparatus that acquires neuropsychological test results. In this case, a testee may answer a neuropsychological test item by using the user terminal 1000, and the user terminal 1000 may transmit the answer result to the server 2000.

The server 2000 may acquire, from the user terminal 1000, answer information corresponding to the testee or a scoring score corresponding to the testee and analyze a cognitive function test result of the testee.

A network may include a local area network (LAN), a wide area network (WAN), a value added network (VAN), a mobile radio communication network, a satellite communication network, and mutual combinations thereof, may be a data communication network in a comprehensive sense, which allows respective network configuration entities shown in FIG. 1 to smoothly communicate with one another, and may include the wired Internet, the wireless Internet, and a mobile wireless communication network. In addition, wireless communication may include, for example, wireless LAN (Wi-Fi), Bluetooth, Bluetooth low energy, ZigBee, Wi-Fi Direct (WFD), ultra wideband (UWB), and infrared data association (IrDA), near field communication (NFC), and the like, but is not limited thereto.

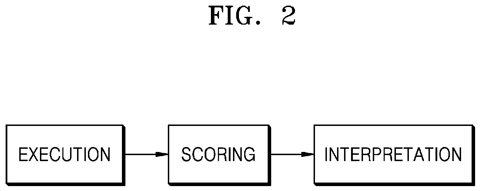

FIG. 2 is a diagram illustrating a neuropsychological test sequence according to an embodiment.

Neuropsychological tests have been developed mainly in the fields of neuropsychiatry, neurology, and neurosurgery to evaluate the status, severity, type, cause, and the like of structural or functional damage to the human brain, and have been used for screening, diagnosis, progress tracking, treatment response evaluation, insurance benefit eligibility evaluation, psychiatric evaluation, and the like of various mental disorders and neurological diseases.

As shown in the example of FIG. 2, a neuropsychological test may be performed in the order of execution, scoring, and interpretation. First, in the execution operation, a testee directly answers a test item. Meanwhile, the neuropsychological test may be executed by providing a questionnaire to the testee or having the testee solve a test item of the neuropsychological test by using the user terminal 1000.

Also, in the scoring operation, the result of performing the test item by the testee is acquired. For example, when a test item is "Memorize and speak 10 words," and the testee speaks 5 words, the results of performing the test item may be "5 pieces", and a scorer or the server 2000 may score a score of the testee corresponding to the corresponding test item as "5 points". Next, the server 2000 performs interpretation on the basis of the scoring score.

Meanwhile, the execution and scoring of the neuropsychological test may also be performed by a semi-expert according to established guidelines. However, the interpretation operation may need a determination of an expert with specialized knowledge in functional neuroanatomy, cognitive psychology, clinical psychology, and the like and experience in performing and interpreting neuropsychological tests. A clinical psychologist is a representative expert, and the Ministry of Health and Welfare manages a qualification of the clinical psychologist through training courses and exams.

However, when direct interpretation by the clinical psychologist is performed every neuropsychological test, the rapidly increasing demand for tests may not be satisfied. In particular, as the number of patients with brain diseases such as dementia and stroke is rapidly increasing due to rapid aging, the number of clinical psychologists may be insufficient compared to the rapidly increasing demand for neuropsychological tests.

Accordingly, a significant portion of the collection of neuropsychological tests executed in hospitals and clinics across the country and most of the collection of neuropsychological tests executed in dementia care centers are executed by semi-experts trained through short-term training courses on execution and scoring, but in this case, appropriate interpretation of results may not be easy.

As an example, some variation may occur according to the compositions and types of subtests of the neuropsychological test, but the execution and scoring operations take 90 to 120 minutes, and interpretation takes about 50 to 70 minutes. In other words, even for a professional clinical psychologist, the interpretation operation accounts for 40 % of the total neuropsychological test processing time, and thus, the lack of clinical psychologists is a major factor in the backlog in neuropsychological test performance.

Accordingly, in an embodiment, a test method capable of accurately interpreting a test result of a testee even when a clinical psychologist does not directly execute a neuropsychological test is described.

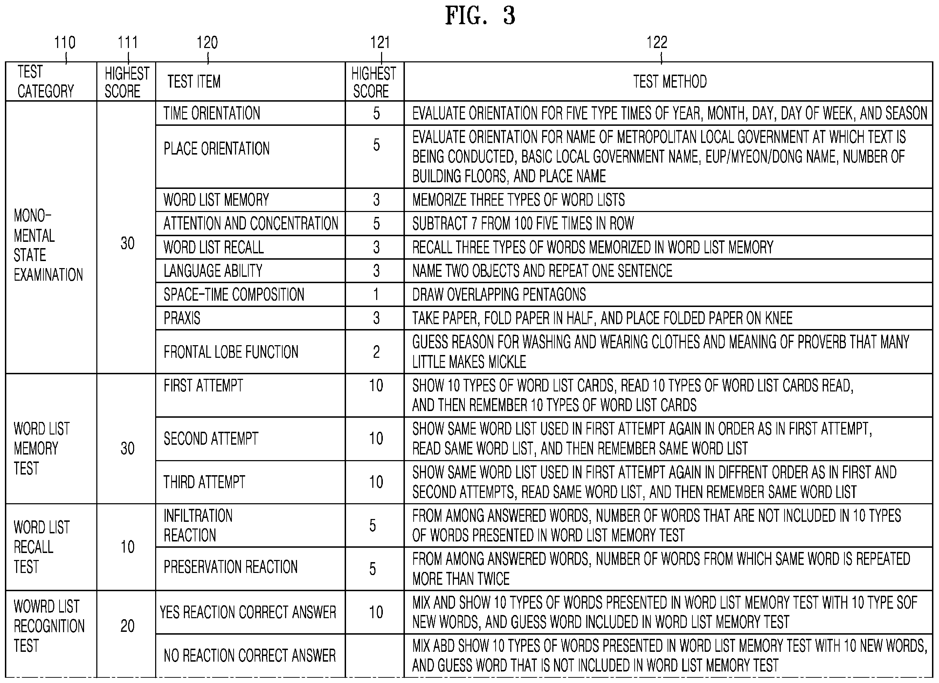

FIG. 3 is a view illustrating an evaluation table including a plurality of test categories and a plurality of test items included in the test categories, according to an embodiment.

The example of FIG. 3 illustrates an evaluation table acquired by the server 2000 for performing a neuropsychological test. According to an embodiment, the server 2000 may acquire one or more test items 120 and one or more test categories 110 including one or more test items.

In an example, the evaluation table may include the test category 110, a highest score 111 of the test category 110, the test item 120, a highest score 121 of the test item 120, and a test method 122 of the test item 120. FIG. 3 is only an example of the evaluation table for easily describing the present disclosure, and the test category 110 and the test item 120 may be added without limitation.

The test category 110 is obtained by categorizing similar test items 120. Also, the highest scores 111 and 121 may indicate the highest scores of evaluation scores of the respective test categories 110 or the test items 120. A testee and a tester, or the testee and the user terminal 1000 may execute a test of each test item 120 with reference to the test method 122.

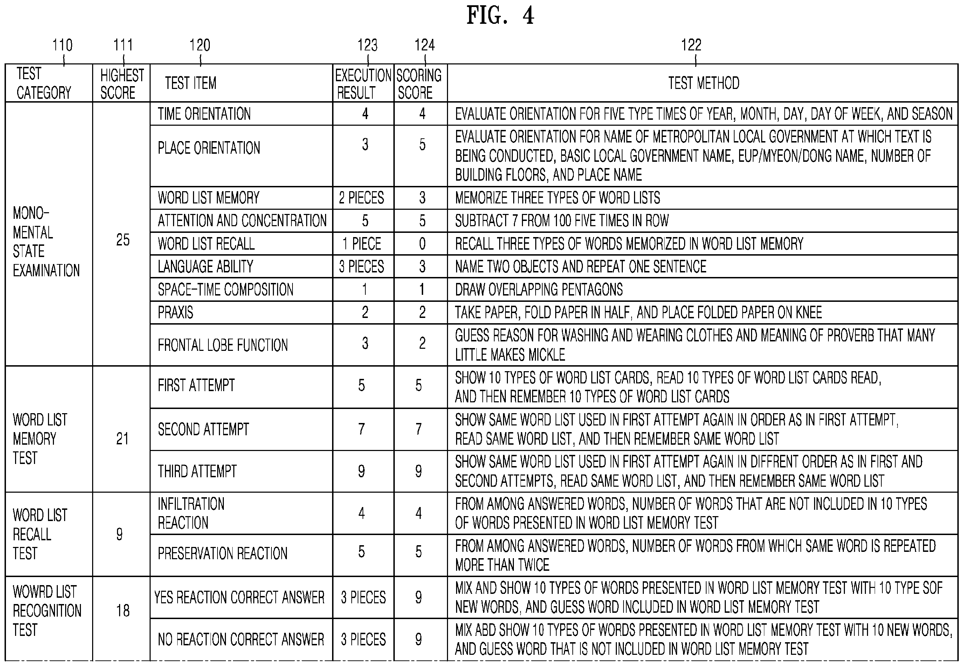

FIG. 4 is a view illustrating converting an execution result of a test item into a scoring score, according to an embodiment.

According to an embodiment, a scorer may input an execution result of a neuropsychological test for a testee by using the user terminal 1000. Here, test items may include items whose execution results directly become scoring scores and which do not need scoring, and items for which the scorer needs to determine and input scoring scores with viewing execution results and thus which need scoring. For example, in the example of FIG. 4, in the case of a time orientation test item that is a test item needing scoring, the scorer may determine an execution result of the testee and give 4 points, and in this case, the scoring score is also equally 4 points. In other words, for an item needing scoring, an execution result input by the scorer may be unchangeably a scoring score.

In another example, the server 2000 may convert, into a scoring score 124 on the basis of a preset criterion, an execution result 123 acquired for an item that does not need scoring. For example, in the example of FIG. 4, for a word list memory test item that does not need scoring, the scorer may input, as an execution result, the number of words memorized by the testee as 2 pieces, and the server 2000 may convert 2 pieces, which are the input execution result, into 3 points, which are a scoring result, on the basis of a preset criterion. Here, the preset criterion for converting the execution result 123 into the scoring score 124 may be determined by the server 2000 or a tester, but is not necessarily limited thereto.

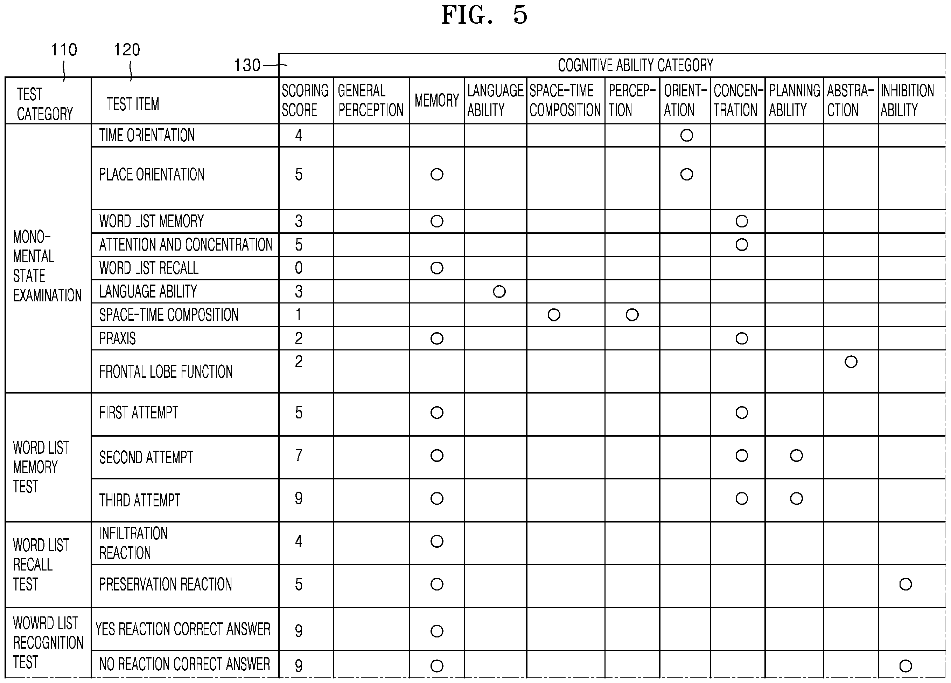

FIG. 5 is a view illustrating matching a test item to a cognitive ability category measured by the corresponding item, according to an embodiment.

In an example, the server 2000 may match each of one or more test items to one or more of a plurality of cognitive ability categories. A cognitive ability category is a category that classifies cognitive functions by type, such as overall cognition, memory, language ability, composition of time and space, perception, orientation, concentration, planning ability, abstraction, and inhibition. According to an example, a test category 110 and a cognitive ability category 130 may match, may not match, or may only partially match.

In an embodiment, the server 2000 may reconstruct a test result of a testee by cognitive function, by matching a test item to a cognitive ability category. In the example of FIG. 5, place orientation in a test item 112 may match memory and orientation. In other words, the place orientation may be determined to be related to the memory and the orientation in a cognitive ability.

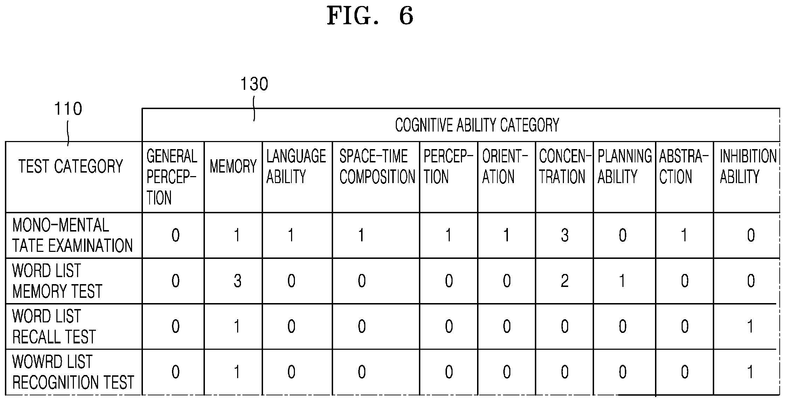

In an example, each of test items may match a plurality of cognitive ability categories. In other words, in an example of FIG. 6, a time orientation test item only matches orientation, but a place orientation may also match memory and orientation. Accordingly, rather than providing a test result corresponding to a simple test item, a test result for each item may be reflected in a complex way to perform an evaluation for each cognitive ability category with high accuracy.

In an embodiment, the server 2000 may calculate an evaluation score for each of a plurality of cognitive ability categories, on the basis of scoring scores of one or more test items matching each of the plurality of cognitive ability categories matching one or more cognitive ability categories.

In an example, the server 2000 may calculate an evaluation score by adding up scoring scores of one or more test items that match a cognitive ability category. For example, referring to FIGS. 4 and 5 together, in the cognitive ability category 130, an evaluation score for a language ability may be 3 points, and an evaluation score for orientation may be 9 points (4 points for time orientation + 5 points for place orientation).

FIG. 6 is a view illustrating reflecting a weight of each test category in an evaluation score, according to an embodiment.

In an example, the server 2000 may acquire a weight of each of test categories for each cognitive ability category, and on the basis of a weight of a test category to which a test item belongs, may calculate, as the evaluation score for each cognitive ability category, a value obtained by reflecting the weights in scoring scores for test items and adding up the scoring scores. In other words, the server 2000 may reflect a significant degree of each test category in the cognitive ability category, by using the weight.

In more detail, as illustrated in the example of FIG. 6, the server 2000 may acquire a weight of a test category 110 for each cognitive ability category 130. In the example of FIG. 6, from among the cognitive ability categories 130, the test category 110 corresponding to memory may include a mono-mental state examination, a word list memory test, a word list recall test, and a word list recognition test, and weights of respective categories may be 1, 3, 1, and 1. In an example, referring to FIGS. 5 and 6 together, a total score of the mono-mental state examination corresponding to the memory category is 10 points (5+3+0+2), and a total score of the word list memory test is 21 points (5 +7+9), a total score of the word list recall test is 9 points (4+5), and a total score of the word list recognition test is 18 points (9+9). In this case, the server 2000 may calculate, as 100 points (10*1+21*3+9*1+18*1), a memory score reflecting the weight.

Next, the server 2000 may determine a level of cognitive impairment on the basis of a scoring score for each cognitive ability category and generate an interpretation statement corresponding to the level of the cognitive impairment. In more detail, the server 2000 may classify the level of the cognitive impairment into three levels, i.e., normal, mild cognitive impairment, and dementia, on the basis of an evaluation score. Also, the server 2000 may generate an interpretation statement made in sentences. In particular, according to an embodiment of the present disclosure, the server 2000 may generate a cognitive ability test interpretation statement by using only a scoring score for each cognitive ability category.

In another embodiment, the server 2000 may acquire one or more pieces of disease information on the basis of the evaluation score for each cognitive ability category and generate disease information on the basis of the one or more pieces of disease information. In an example, according to the disease information, presumed cause diseases of cognitive impairment may be categorized into Alzheimer's disease and related diseases, frontotemporal dementia, semantic dementia, progressive non-fluent aphasia, depression, and others. The server 2000 may generate an interpretation statement made in sentences, by using matching disease information.

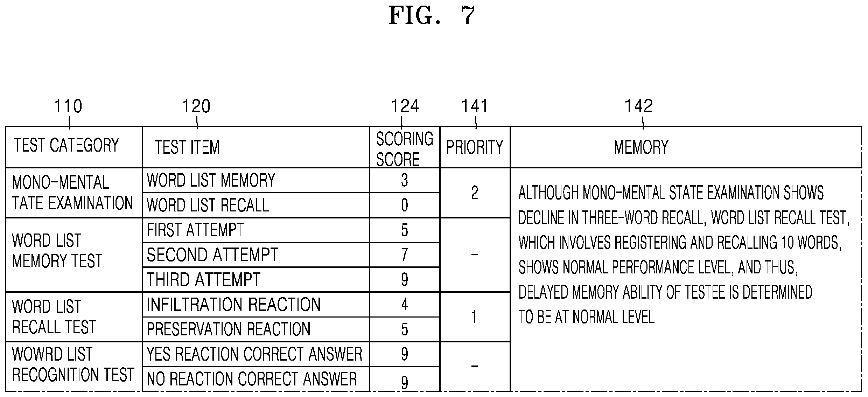

FIG. 7 is a view illustrating generating an interpretation statement on the basis of priority, according to an embodiment.

According to an embodiment, the server 2000 may generate an interpretation statement on the basis of priorities of test items. In an example, when a testee answers a test item, answers, which conflict with each other, may occur. The answers, which conflict with each other, may include cases in which answers to the test items are inconsistent or irrational. The server 2000 may determine answers that conflict with each other, on the basis of a preset criterion. In this case, the answers, which conflict with each other, are highly likely to be noise, and thus, the server 2000 may correct an execution score by reflecting the same. In other words, noise may be prevented from being mixed into a test result by generating an interpretation statement on the basis of priorities of test items.

In a detailed example, the server 2000 may determine a priority of a test category for each cognitive ability category. FIG. 7 illustrates priorities among test categories 110, with respect to memory in a cognitive ability category. Here, when a priority 141 has a smaller number, the priority 141 may be a category. According to the example of FIG. 7, with respect to memory, a priority of a word list recall test may be higher than a priority of a mono-mental state examination. In other words, even when a scoring score for the mono-mental state examination is low, a scoring score for the word list recall test may be ignored when a score of the word list recall test is high. Accordingly, the server 2000 may generate, on the basis of the priority 141, an interpretation statement for the memory as "Although a mono-mental state examination shows a decline in three-word recall, a word list recall test, which involves registering and recalling 10 words, shows a normal performance level, and thus, a delayed memory ability of a testee is determined to be at a normal level".

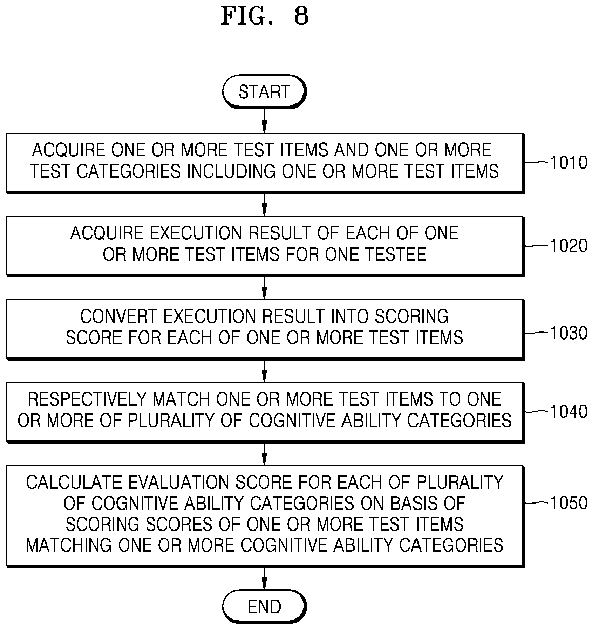

FIG. 8 is a flowchart sequentially illustrating a neuropsychological test method according to an embodiment.

First, in operation 1010, the server 2000 acquires one or more test items and one or more test categories including one or more test items.

Also, in operation 1020, the server 2000 acquires an execution result of each of one or more test items for one testee.

In addition, in operation 1030, the server 2000 converts the execution result into a scoring score for each of the one or more test items.

In operation 1040, each of the one or more test items is matched to one or more of a plurality of cognitive ability categories.

In operation 1050, on the basis of scoring scores for the one or more test items matching each of the plurality of cognitive ability categories, an evaluation score for each of the plurality of cognitive ability categories is calculated.

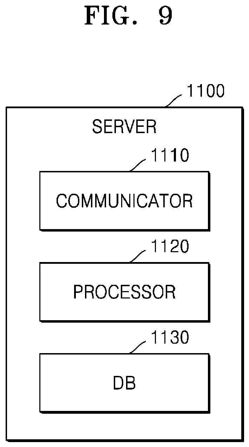

FIG. 9 is a block diagram of a server according to an embodiment.

A server 1100 of FIG. 9 may be a game server 2000.

Referring to FIG. 9, the server 1100 may include a communicator 1110, a processor 1120, and a DB 1130. The server 1100 of FIG. 9 illustrates only components related to an embodiment. Therefore, it may be understood by one of ordinary skill in the art that other general-purpose components may be further included in addition to the components shown in FIG. 9.

The communicator 1110 may include one or more components that enable wired/wireless communication with other nodes. For example, the communicator 1110 may include at least one of a short-range communicator (not shown), a mobile communicator (not shown), and a broadcast receiver (not shown).

The DB 1130 may be hardware for storing various types of data processed within the server 1100, and may store programs for processing and controlling by the processor 1120. DB 1130 may store payment information, user information, and the like.

The DB 1130 may include random access memory (RAM), such as dynamic random access memory (DRAM) and static random access memory (SRAM), read-only memory (ROM), electrically erasable programmable read-only memory (EEPROM), CD-ROM, Blu-ray or other optical disk storages, a hard disk drive (HDD), a solid state drive (SSD), or flash memory.

The processor 1120 controls an overall operation of the server 1100. For example, the processor 1120 may generally control an input unit (not shown), a display (not shown), the communicator 1110, the DB 1130, and the like by executing the programs stored in the DB 1130. The processor 1120 may control an operation of the server 1100 by executing the programs stored in the DB 1130. described above with reference to FIGS. 1 to 8.

The processor 1120 may be implemented by using at least one of application specific integrated circuits (ASICs), digital signal processors (DSPs), digital signal processing devices (DSPDs), programmable logic devices (PLDs), field programmable gate arrays (FPGAs), controllers, micro-controllers, microprocessors, and electrical units for performing other functions.

Embodiments according to the present disclosure may be implemented in the form of a computer program that may be executed on a computer via various types of components, and the computer program may be recorded on a computer-readable medium. Here, the medium may include magnetic media, such as a hard disk, a floppy disk, and a magnetic tape, optical recording media, such as CD-ROM and DVD, a magneto-optical medium, such as a floptical disk, and hardware devices, such as ROM, RAM, and flash memory devices specially configured to store and execute program instructions.

Meanwhile, the computer program may be specially designed and configured for the present disclosure, or may be known to and used by those skilled in the art of the computer software field. Examples of the computer program may include not only machine language code generated by a compiler but also high-level language code that may be executed by a computer by using an interpreter or the like.

According to one embodiment, the method according to various embodiments of the present disclosure may be included and provided in a computer program product. The computer program product may be traded between a seller and a buyer as a product. The computer program product may be distributed in the form of a device-readable storage medium (e.g., compact disc read only memory (CD-ROM)), or may be distributed (e.g., downloaded or uploaded) online via an application store (e.g., Play Store TM) or directly between two user devices. When distributed online, at least a portion of the computer program product may be at least temporarily stored or temporarily generated in a device-readable storage medium such as a server of a manufacturer, a server of an application store, or a memory of a relay server.

## Claims

1. A method of interpreting neuropsychological test results, the method comprising:
an operation of acquiring one or more test items and one or more test categories including the one or more test items;
an operation of acquiring an execution result of each of the one or more test items for one testee;
an operation of converting the execution result into a scoring score for each of the one or more test items;
an operation of matching each of the one or more test items to one or more of a plurality of cognitive ability categories;
an operation of calculating an evaluation score for each of the plurality of cognitive ability categories, on the basis of scoring scores of the one or more test items matching each of the plurality of cognitive ability categories; and
an operation of generating a cognitive ability interpretation result on the basis of the calculated evaluation score.

2. The method of claim 1, wherein the operation of calculating the evaluation score comprises calculating the evaluation score by adding up all the scoring scores of the one or more test items matching the cognitive ability categories.

3. The method of claim 1, wherein the operation of calculating the evaluation score comprises acquiring a weight of the test category for each of the cognitive ability categories, and on the basis of the weight of the test category to which the test items belong, calculating, as the evaluation score for each of the cognitive ability categories, a value obtained by reflecting the weight in the scoring scores of the test items and adding up the scoring scores.

4. The method of claim 1, wherein the operation of generating the cognitive ability interpretation result comprises determining a level of cognitive impairment on the basis of the evaluation score for each of the cognitive ability categories and generating an interpretation statement corresponding to the level of the cognitive impairment.

5. The method of claim 4, wherein the operation of generating the cognitive ability interpretation result comprises acquiring one or more pieces of disease information on the basis of the evaluation score for each of the cognitive ability categories and generating the interpretation statement on the basis of the one or more pieces of disease information.

6. The method of claim 1, wherein the operation of generating the cognitive ability interpretation result comprises:
an operation of acquiring priorities for the respective test categories of the one or more test items; and
an operation of generating the cognitive ability interpretation result on the basis of the priorities of the respective test categories.

7. A server for interpreting neuropsychological test results, the server comprising:
a processor; and
a memory, wherein
the processor is configured to
acquire one or more test items and one or more test categories including the one or more test items,
acquire an execution result of each of the one or more test items for one testee,
convert the execution result into a scoring score for each of the one or more test items,
match each of the one or more test items to one or more of a plurality of cognitive ability categories,
calculate an evaluation score for each of the plurality of cognitive ability categories, on the basis of scoring scores for the one or more test items matching each of the plurality of cognitive ability categories, and
generate a cognitive ability interpretation result on the basis of the calculated evaluation score.

8. A computer-readable recording medium having recorded thereon a program for causing a computer to execute the method of claim 1.
